# EUROPEAN PATENT APPLICATION

(11) **EP 2 540 838 A1**
(43) Date of publication of application: **02.01.2013**
(21) Application number: 11746812.4
(22) Date of filing: 22.02.2011
(51) Int. Cl.: C12Q 1/68, C12N 15/11

(54) **KIT FOR QUANTITATIVE DETECTION OF BRAF MUTATION**

(30) Priority: 24.02.2010 CN 201010113309
(71) Applicant: Beijing ACCB Biotech Ltd., Beijing 100094 (CN)
(72) Inventor: XU, Junpu, Beijing 100094 (CN); CHEN, Zhao, Beijing 100094 (CN); LI, Jun, Beijing 100094 (CN)
(74) Representative: Dossmann, Gérard
(86) International application number: PCT/CN2011/000270
(87) International publication number: WO 2011/103770

(57) **Abstract**

The present invention relates to a method and assay kit for BRAF gene mutations which relates to the effect of molecule-targeting anti-tumor drug. Particularly, the present invention relates to a fluorescent quantitative PCR method and kit for detecting mutations at hotspots of BRAF gene, together with the use thereof. The present invention detects the mutations at specific sites of BRAF gene, and can predict the therapeutic efficacy of anti-EGFR tyrosine kinase inhibitors, an anti-tumor drug. Therefore, the present invention can provide a guidance to individualized treatments for cancer patients.

SEQUENCE LISTING

<110>  Beijing ACCB Biotech Ltd.

<120>  AN ASSAY KIT FOR QUANTITATIVELY DETECTING BRAF GENE MUTATIONS

<130>  MUB12-5060EP

<160>  14

<170>  PatentIn version 3.3

<210>  1
<211>  100
<212>  DNA
<213>  unknown

<220>
<223>  Sequence of BRAF Exon 15

<300>
<308>  NCBI NM_004333

<400>  1
ccatgaagac ctcacagtag taaaaatagg tgattttggt ctagctacag tgaaatctcg     60

atggagtggg tcccatcagt ttgaacagtt gtctggatcc                           100


<210>  2
<211>  150
<212>  DNA
<213>  unknown

<220>
<223>  a part of wild-type DNA sequence of BRAF

<300>
<308>  NCBI NC_000007.12

<400>  2
ctcagatata tttcttcatg aagacctcac agtaaaaata ggtgattttg gtctagctac     60

agtgaaatct cgatggagtg ggtcccatca gtttgaacag ttgtctggat ccattttgtg    120

gatggtaaga attgaggcta tttttccact                                      150


<210>  3
<211>  30
<212>  DNA
<213>  Artificial

<220>
<223>  upstream primer of BRAF

<400>  3
catgaagacc tcacagtaaa aataggtgat                                       30


<210>  4
<211>  24
<212>  DNA
<213>  Artificial

<220>
<223>  upstream primer of BRAF

<400>  4
ttcttcatga agacctcaca gtaa                                              24


<210>  5
<211>  25
<212>  DNA
<213>  Artificial

<220>
<223>  downstream primer of BRAF

<400>  5
ggatccagac aactgttcaa actga                                             25


<210>  6
<211>  23
<212>  DNA
<213>  Artificial

<220>
<223>  downstream primer of BRAF

<400>  6
ccagacaact gttcaaactg atg                                               23


<210>  7
<211>  21
<212>  DNA
<213>  Artificial

<220>
<223>  upstream primer of BRAF Codon 600 GTG->GAG mutation

<400>  7
tacagagaaa tctcgatgga g                                                 21


<210>  8
<211>  21
<212>  DNA
<213>  Artificial

<220>
<223>  downstream primer of BRAF Codon 600 GTG->GAG mutation

<400>  8
atttctctgt agctagacca a                                                 21


<210>  9
<211>  20
<212>  DNA
<213>  Artificial

<220>
<223>  probe of wild-type BRAF Codon 600

<400>  9
ccatcgagat ttcactgtag                                                   20

<210>  10
<211>  28
<212>  DNA
<213>  Artificial

<220>
<223>  wild-type probe of BRAF Codon 600

<400>  10
ccatcgagat ttcactgtag ctagacca                                          28


<210>  11
<211>  20
<212>  DNA
<213>  Artificial

<220>
<223>  probe for BRAF Codon 600 GTG -> GAG mutation

<400>  11
ccatcgagat ttctctgtag                                                   20


<210>  12
<211>  28
<212>  DNA
<213>  Artificial

<220>
<223>  probe for BRAF Codon 600 GTG -> GAG mutation

<400>  12
ccatcgagat ttctctgtag ctagacca                                          28


<210>  13
<211>  26
<212>  DNA
<213>  Unknown

<220>
<223>  DNA sequence containing wild-type sequence of BRAF Codon 600

<400>  13
ggtctagcta cagtgaaatc tcgatg                                            26


<210>  14
<211>  26
<212>  DNA
<213>  Unknown

<220>
<223>  DNA sequence containing BRAF Codon 600 GAG mutant sequence

<400>  14
ggtctagcta cagagaaatc tcgatg                                            26

## Description

### BACKGROUND OF THE INVENTION

BRAF gene belongs to the RAF gene family. It is an oncogene encoding a serine/threonine kinase, an important member of the RAS-RAF-MEK-ERK signal transduction pathway, which plays important roles in regulating cell proliferation, differentiation and apoptosis (Ikenoue T., Cancer Res., 2003, 63(23):8123-37). Therefore, BRAF gene has been implicated in tumorigenesis and tumor development, and it can serve as potential diagnosis marker and therapy target.

BRAF gene locates at the 7q34 site, encoding a protein of 783 amino acids. Many studies have shown that different mutations can occur in the BRAF gene with various ratios in malignant melanoma, colon cancer, lung cancer, thyroid carcinoma, hepatocarcinoma and pancreatic cancers (Davies H. et al., Nature, 2002, 417(6892): 949-54), wherein about 90% BRAF mutations are located at nucleotide No 1799, in which T is replaced with A, so that the encoded amino acid at position 600 changes from glutamine to valine (Wang L. et al., Cancer Res., 2003, 63(17): 5209-12). The present invention uses real-time quantitative PCR to detect the mutation at the codon encoding amino acid at position 600 in BRAF, a tumor-related gene, so as to predict drug resistance to chemotherapy (Di Nicolantonio F. et al., J. Clin. Oncol., 2008, 26(35): 5705-12).

The detecting method of the present invention has the following advantages: easy manipulation, and easy standardization. Other methods, such as allele specific oligonucleotide probe hybridization method, are very much dependent on hybridization conditions, and therefore require strict control of the experimental conditions. The restriction fragment length polymorphism method, on the other hand, needs a lot of human labor, and can not generate quantitative results. The method of the present invention has short experimental cycle, and can be completed within 2 hours. It doesn't need verification of the results by sequencing, whereas the direct sequencing and high resolution melting analysis need 4 days to 2 weeks. Sensitivity of the method of the present invention is high, which, after optimizing experimental conditions, can reach 1% for detecting mutations, whereas sensitivity of direct sequencing is 20-50%. Specificity of the method of the present invention is also high. Immunohistochemistry (IHC) method can easily get pseudo-positive and pseudo-negative results, and can not determine the position and types of point mutations. The unique advantage of the present invention is accurate quantification. By using absolute quantification method to analyze data, draw standard curve, and accurately determine the content of wild-type gene and mutant gene in the samples, one can obtain ratio of the mutant gene in the samples, which will be helpful for clinical diagnosis and therapeutic selection. Furthermore, the present invention is safe and non-toxic, other methods such as chemical breaking method of mismatched base pairs need isotope and toxic chemical agents.

### SUMMARY OF THE INVENTION

The question that the present invention addresses is to provide an assay kit for quantitatively detecting an BRAF gene mutation, which can quantitatively detect the following mutations: GTG, the codon encoding amino acid at position 600 in BRAF gene, is replaced with GAG.

To address the above question, the present invention provides quantitative detection kit containing a mixture comprising Taq enzyme, 10 x Taq buffer, MgCl₂, dNTP mixture, PCR primers which can specifically amplify the sequences at BRAF gene mutation positions, and probes which can specifically identify wild-type sequences and mutant sequences, together with method of the detection, as follows:
(1) Separately design upstream and downstream primers around the mutation positions of Codon 600 of BRAF gene; and design specific probes according to each mutant site. Said probes can specifically bind wild-type sequences or the mutant sequences to be detected at specific BRAF sites, so as to determine whether the tested mutations occur at said sites.
(2) To accurately and quantitatively determine the ratio of the BRAF mutations, standards were designed in the present invention.
(3) Use fluorescent quantitative PCR to detect the samples and standards.
(4) Obtain standard curves for quantitative detection from the detection results of the standards, and calculate the ratios of BRAF gene mutations to the total wild type BRAF gene in the samples to be tested.

Prior to said step (1) it further includes: extracting nucleic acid from the samples, purifying it and determining the content of it.

The probes for fluorescent quantitative PCR specifically bind the sequences at BRAF gene mutation sites under suitable PCR conditions. Preferably, said probes link a fluorescence emitting group at their 5' end, and link a fluorescence quenching group at their 3' end. Said fluorescence emitting group is selected from FAM, TET, HEX and ROX. Said fluorescence quenching group is selected from BHQ, TAMARA. Preferably, said emitting group is FAM, and said quencher group is BHQ. Preferably, the sequences of said probes are selected from the group consisting of SEQ ID NO: 9, SEQ ID NO: 10, SEQ ID NO: 11 and SEQ ID NO: 12.

Said standards include at least one of plasmids, genome DNA or chemically synthesized sequences. Preferably, said standards comprises a wild-type plasmid, a mutant plasmid, or both a wild-type plasmid and a mutant plasmid, wherein said wild-type plasmids include wild-type sequences of BRAF gene, and said mutant plasmids include mutant sequences of BRAF gene. More preferably, said standards are consisted of a wild-type plasmid, a mutant plasmid, or both a wild-type plasmid and a mutant plasmid.

The tested samples include fresh tissue, paraffin embedded tissues, cell lines, blood, pleural effusion, peritoneal effusion, saliva, digestive juice, urine and feces.

Said primers consist of upstream primers and downstream primers. Preferably, said primers are selected from the group consisting of SEQ ID NO: 3, SEQ ID NO: 4, SEQ ID NO: 5, and SEQ ID NO: 6.

Said quantitative detection kit for BRAF gene mutations includes the agents selected from: the above-mentioned primers, probes and standards. Preferably, said kit further includes Taq enzyme, 10 x Taq buffer, MgCl₂, and dNTP mixture. Preferably, the ratio of said primer to probe is 2:1-10:1, and said primer comprises a forward primer and reverse primer in a ratio of 1:3-3:1. Said standards include a mixture of said plasmids in a certain ratio, wherein the ratio of the content of wild-type plasmids to mutant plasmids is 0%-100%.

### BRIEF DESCRIPTION OF DRAWINGS

The accompanying drawings, which are incorporated in and form a part of this specification, illustrate embodiments of the technology and together with the description.
Figure 1 is a diagram showing the method for constructing the plasmid standards in Example 2.
Figure 2 is a diagram showing the wild-type plasmid profile of Example 2, wherein the wild-type PCR product sequence is inserted into the carrier at the position marked with an arrow.
Figure 3 is a diagram showing the result of sequencing the wild-type plasmid standard of Example 2.
Figure 4 is a diagram showing the result of sequencing the mutant plasmid standard of Example 2, wherein the mutant site is marked with an arrow.
Figure 5 is a diagram showing the amplification curve of the standard of Example 3, wherein Fig.A is the amplification curve of BRAF wild-type plasmid, Fig.B is the amplification curve of BRAF Codon 600 GTG**→** GAG mutant plasmid.
Figure 6 is a diagram showing the standard curve based on Figure 4, wherein Fig.A is the standard curve of BRAF wild-type plasmid, Fig.B is the standard curve of BRAF Codon 600 GTG**→**GAG mutant plasmid.
Figure 7 shows the amplification curve of fluorescent quantitative PCR of the wild-type (Fig.A) and GTG**→**GAG mutant (Fig.B) of BRAF Codon 600 in a paraffin embedded tissue sample tested in Example 3; wild-type (Fig.C) and GTG**→**GAG mutant (Fig.D) of BRAF Codon 600 in a fresh tissue sample; wild-type (Fig.E) and GTG**→**GAG mutant (Fig.F) of BRAF Codon 600 in whole blood sample; and wild-type (Fig.G) and GTG**→**GAG mutant (Fig.H) of BRAF Codon 600 in cell line sample.

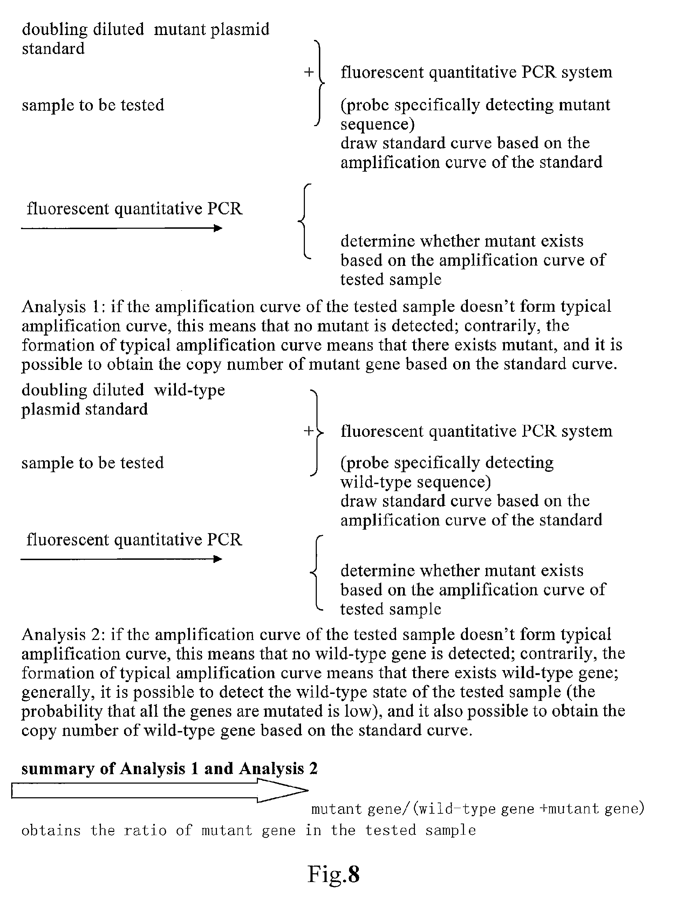

Figure 8 is a diagram of the quantitative method of the present invention.

### DETAILED DESCRIPTION OF PARTICULAR EMBODIMENTS

### Examples

The following examples are put forth so as to provide those of ordinary skill in the art with a complete disclosure and description of how to make use of the present invention, and are neither intended to limit the scope of what the inventors regard as their invention nor are they intended to represent that the experiments below are all or the only experiments performed. The experimental conditions not indicated in the Examples, are generally conventional, such as those disclosed in "Molecular Cloning, A Laboratory Manual, 3rd ed, (Sambrook J.)", or those suggested by the manufacturer.

### Example 1: Extracting genome DNA from fresh human tumor tissues, paraffin embedded tissues, peripheral blood, pleural effusion, and human cell lines

The tumor cell lines we tested included cell lines of: non-small-cell lung carcinoma (NSCLC; A549, H460, H838 and H1703), breast cancer (MCF-7, BT474 and HuL100), malignant mesothelioma (H513, H2052, H290, MS-1 and H28), thyroid carcinoma(KAT10), colon cancer (SW480, S1-M1-80), head and neck cancer (U87), cervical carcinoma (Hela), sarcoma (Mes-SA, Saos-2 and A204).

The fresh human tumor tissues, peripheral blood, paraffin embedded tissues we tested included: NSCLC, mesothelioma, colon cancer, malignant melanoma, renal carcinoma, esophagus cancer, thyroid carcinoma, malignant cancer and ovarian cancer.

### Extraction of sample DNA

DNA extracting kit from Qiagen Inc., Promega Inc., or Roche Inc. can be used to extract genomic DNA from the samples. Content and purity of the extracted DNA can be determined by using Nanodrop ND1000 (Gene Inc.) (OD260/OD280 is about 1.8, OD260/OD230 is more than 2.0). For example, the sample DNA may be extracted using the DNA Extracting Kit (Promega Inc.) as follows:
1. DNA extraction from fresh tissues
   (1) cut a bean-sized tissue using scissors, put it into a mortar, cut it into pieces, and ground it into powder by adding liquor nitrogen.
   (2) add 600µl pre-cooled lysate into the mortar, blow it 6 times using 1ml tip, sufficiently mix the tissue powder and the lysate, transfer the mixture into a 1.5ml EP tube, then turn it over 6 times, water bath under 65°C for 20 minutes.
   (3) add 3µl RNase, turn over 6 times to mix homogenously, water bath under 37°C for 20 minutes.
   (4) cool it to room temperature, add 200µl protein precipitation agent, turn over 6 time to mix homogenously, place it on ice for 5 minutes, 13000 x g centrifuge 4 minute at room temperature.
   (5) transfer the supernatant into a new EP tube pre-added with 600µl isopropanol (room temperature), gently mix 6 times, then 13,000 x g centrifuge at room temperate for 1 minutes.
   (6) discard the supernatant, add 600µl 70% ethanol (room temperature) into precipitate, 13,000 x g centrifuge at room temperate for 1 minutes.
   (7) aspirate out ethanol, air dry for 15 minutes.
   (8) add 40µl DNA dissolving solution into the precipitate, incubate at 65°C for 1 hour or 4°C overnight.
2. DNA extraction from paraffin embedded tissues
   (1) add 1 mg or less tissues into 1.5ml centrifuge tube.
   (2) add freshly prepared 100µl incubation buffer/proteinase K solution, and incubate at 56°C overnight based on the type of the samples.
   (3) take out the incubated sample tube, add two times volume of lysate buffer.
   (4) vortex-oscillate the resin for 10 seconds until the resin is fully suspended, add 7µl fully suspended resin, vortex-oscillate the resin for 3 seconds, then incubate at room temperature for 5 minutes.
   (5) vortex-oscillate the resin for 2 seconds, put the tube on a magnetic separation rack (MagneSphere®), immediately conduct magnetic separation.
   (6) carefully remove all solution, without touching the resin on the tube wall.
   (7) add 100µl lysate buffer, remove the tube from the magnetic separation rack, vortex-oscillate for 2 seconds.
   (8) put the tube back to the magnetic separation rack, remove all the lysate.
   (9) add 100µl 1x washing fluid, remove the tube from the magnetic separation rack, vortex oscillate 2 seconds.
   (10) put the tube back to the magnetic separation rack, remove all the lysate.
   (11) repeat step (9) and (10) twice, totally wash three times, and remove all the liquid after the last wash.
   (12) open the lid, put the tube on the magnetic separation rack, air dry for 5 minutes.
   (13) add 25µl eluate.
   (14) close the lid, vortex oscillate for 2 seconds, incubate at 65°C for 5 minutes.
   (15) take out the incubated tube, vortex oscillate for 2 seconds, immediately put it on the magnetic separation rack.
   (16) carefully transfer the DNA solution into a selected container.
3. DNA extraction of whole blood
   (1) obtain 300µl anticoagulant whole blood, add 900µl cell lysate, blow 6 times using 1ml tip, so that the whole blood and the cell lysate are sufficiently mixed, place it under room temperature for 10 minutes, blow with the tip three times.
   (2) 13,000 x g centrifuge under room temperature for 20 seconds, discard the supernatant, shake violently, add 300µl pre-cooling lysate, blow with 1ml tip until the precipitate are totally dissolved.
   (3) add 1.5µl RNase, turn over 6 times to mix homogenously, water bath under 37°C for 20 minutes.
   (4) cool to room temperature, add 100µl protein precipitation agent, turn over 6 times to mix homogenously, place it on ice for 5 minutes, 13,000 x g centrifuge under room temperature for 4 minutes.
   (5) transfer the supernatant to a new EP tube previously added 300µl isopropanol (room temperature), gently mix 6 times, centrifuge under room temperature for 1 minutes.
   (6) discard the supernatant, add 1 ml 70% ethanol (room temperature) into the precipitate, turn over 6 times to mix homogenously, 13,000 x g centrifuge under room temperature for 1 minutes.
   (7) aspirate out ethanol, air dry for 15 minutes.
   (8) add 40µl DNA dissolving solution, stay at 65°C for 1 hour or 4°C overnight.
4. DNA extraction of pleural effusion
   (1) obtain 5 ml pleural effusion, 2000 rpm centrifuged at room temperature for 10 minutes, remove the supernatant, add 1 ml cell lysate, turn over 6 times to mix homogenously, stay under room temperature for 10 minutes.
   (2) 13,000 x g centrifuged under room temperature for 20 seconds, discard the supernatant, shake violently, add 1 ml pre-cooling lysate, mix until the precipitate totally dissolved.
   (3) add 3 µl RNase, turn over 6 times to mix homogenously, water bath under 37°C for 20 minutes.
   (4) cool to room temperature, add 200µl protein precipitation agent, turned over 6 times to mix homogenously, place it on ice for 5 minutes, 13,000 x g centrifuged under room temperature for 4 minutes.
   (5) transfer the supernatant to a new EP tube previously added 5 ml isopropanol (room temperature), gently mix 6 times, 13,000 x g centrifuged under room temperature for 1 minutes.
   (6) discard the supernatant, add 1 ml 70% ethanol (room temperature) into the precipitate, turn over 6 times to mix homogenously, 13,000 x g centrifuged under room temperature for 1 minutes.
   (7) aspirate out ethanol, air dry for 15 minutes.
   (8) add 40µl DNA dissolving solution, stay at 65°C for 1 hour or 4°C overnight.
5. DNA extraction from cell lines
   (1) obtain at least 1 x 10⁶ cells, transfer them into a 1.5 ml EP tube, 13,000 x g centrifuged at room temperature for 10 seconds. If the cells are adherent cells, they should be digested by trypsin before collecting them.
   (2) discard the supernatant, add 200 µl PBS to wash the cells, 13,000 x g centrifuged under room temperature for 10 seconds, discard the supernatant, shake violently until the precipitate is suspended.
   (3) add 600 µl pre-cooling lysis solution, blow to mix homogenously with 1 ml tip until no visual cell blocks.
   (4) add 3 µl RNase, turn over 6 times to mix homogenously, water bath under 37°C for 20 minutes.
   (5) cool to room temperature, add 200µl protein precipitation agent, turn over 6 times to mix homogenously, place it on ice for 5 minutes, 13,000 x g centrifuged under room temperature for 4 minutes.
   (6) transfer the supernatant to a new EP tube previously added 600µl isopropanol (room temperature), gently mix 6 times, 13,000 x g centrifuged under room temperature for 1 minutes.
   (7) discard the supernatant, add 600 µl 70% ethanol (room temperature) into the precipitate, turn over 6 times to mix homogenously, 13,000 x g centrifuged under room temperature for 1 minutes.
   (8) aspirate out ethanol, air dry for 15 minutes.
   (9) add 40µl DNA dissolving solution, stay at 65°C for 1 hour or 4°C overnight.

### Example 2: Preparation of the plasmid standards containing mutant and wild-type sequences

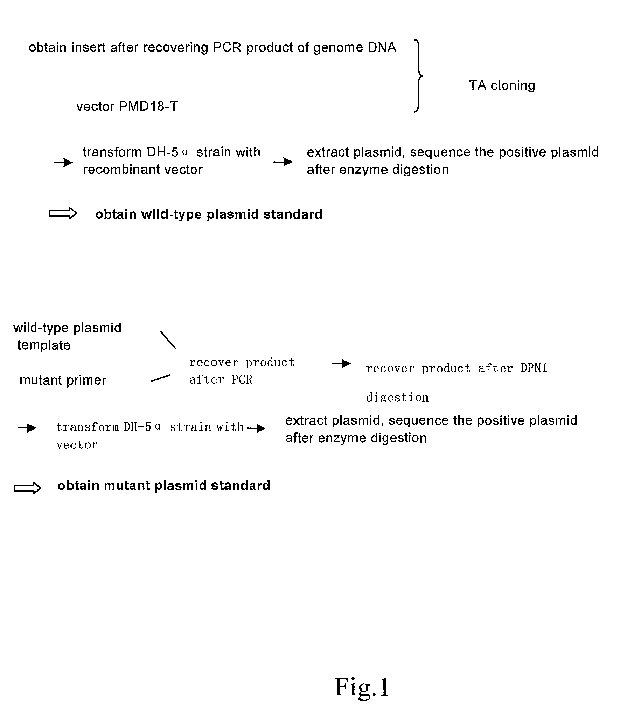

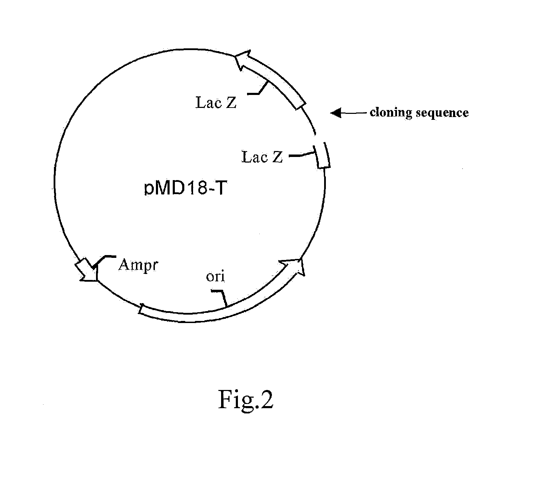

### 1. Construction of wild-type plasmids (Figure 1, Figure 2)

### 1.1 Preparation of the carrier

TA cloning carrier pMD 18-T was purchased from TAKARA Inc.

### 1.2 Preparation of the insert

The insert is prepared using PCR. The template of PCR is the sample genome DNA extracted in Step 1. The reaction system and amplification condition are shown in the following tables (Table 1, Table 2 and Table 3):

**Table 1: PCR reaction system (50µl)**

| reagents | amount(µl/tube) |
|---|---|
| double-distilled water | 29.75 |
| 10x buffer (free of Mg²⁺) | 5 |
| MgCl₂ (25mM) | 7.5 |
| dNTP (10 mM) | 1.25 |
| upstream primer (25µM) | 1.25 |
| downstream primer (25 µM) | 1.25 |
| Taq enzyme | 1 |
| DNA template | 3 |
| total volume | 50 |

**Table 2: PCR primers**

| name | Sequence | |
|---|---|---|
| BRAF -F₁ | CATGAAGACCTCACAGTAAAAATAG GTGAT | (SEQ ID NO:3) |
| BRAF -F₂ | TTCTTCATGAAGACCTCACAGTAA | (SEQ ID NO:4) |
| BRAF -R₁ | GGATCCAGACAACTGTTCAAACTGA | (SEQ ID NO:5) |
| BRAF -R₂ | CCAGACAACTGTTCAAACTGATG | (SEQ ID NO:6) |

**Table 3: PCR amplification condition**

| step | cycles | temperature and time |
|---|---|---|
| step 1 | 1 | 95°C, 1-5 minutes |
| step 2 | 20-30 | 95°C, 10-15 seconds; 55-65°C, 30-60 seconds |

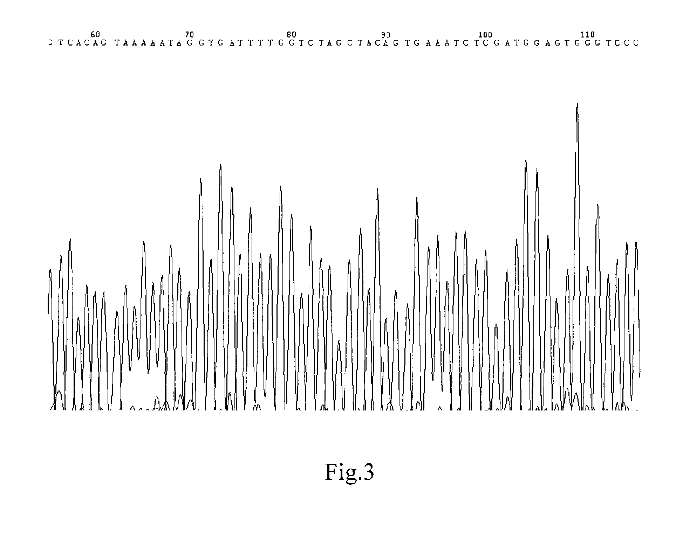

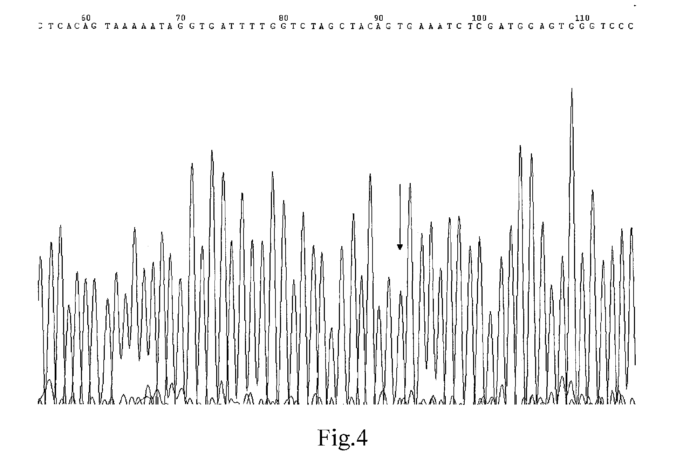

1.3 After recovering the target fragment using QIAgen Gel Recover Kit, insert said fragment into pMD18-T (purchased from TAKARA Inc.) by TA colonizing.
1.4 Amplify the constructed plasmid in E. coli DH5α strain, and harvest by extraction and purification (the methods are showed in Molecular Cloning, A Laboratory Manual, 3rd ed. pages 96-99 and 103.
1.5 Identify the plasmid by double enzyme digestion of BamHI and HindIII.
1.6 Sequence the strains having positive result, and use the strains with correct sequence as the standard containing wild-type sequence (Figure 3).
2. Construction of mutant plasmids: design mutant primers of mutant sites, obtain the standards containing mutant sequences by DPN1 method.
2.1 Design the mutant primers (Figure 4) of mutant sites based on the desired mutant sequences.

**Table 4: mutant primers**

| primers name | sequences | |
|---|---|---|
| BRAF-1-F: | TACAGAGAAATCTCGATGGAG | (SEQ ID NO:7) |
| BRAF-1-R: | ATTTCTCTGTAGCTAGACCAA | (SEQ ID NO:8) |

2.2 Use 5ng wild-type plasmid as template, and use mutant primers and Pfu enzyme to mutate the target sites. The amplification system and condition are shown in Table 1, Table 4 and Table 3.
During the preparation of the plasmid containing BRAF Codon 600 GTG **→**GAG mutant sequence, BRAF-1-F (SEQ ID NO:7) and BRAF-1-R (SEQ ID NO:8) primers are needed to add into the amplification system.
2.3 treat the product obtained in step 2.2 with DPN1 enzyme, recover the product after incubating at 37°C for 1 hour, amplify in E. coli DH5α strain, and harvest by extraction and purification.
2.4 Identify the plasmid by double enzyme digestion of BamHI and HindIII.
2.5 Sequence the strains having positive result, and use the strains with correct sequence as the standard containing mutant sequence (Figure 4).

### Example 3: Detection of BRAF mutations from genome DNA of human cell lines, human fresh tumor tissues, peripheral blood, and paraffin embedded tissues, using samples of thyroid carcinoma and colon cancer as examples.

1. The templates for fluorescent quantitative PCR are the genome DNA of thyroid carcinoma and colon cancer samples extracted in Example 1, and the standards prepared in Example 2. Double-distilled water is served as negative control. For drawing the standard curves, the standards are diluted as 1ng/µl 0.5ng/µl 0.25ng/µl 0.125ng/µl 0.0625ng/µl 0.03125ng/µl.
2. The reaction system and condition are shown in Table 2, Table 5, Table 6 and Table 7, wherein the fluorescent emission group bound to the probe is selected from FAM, TET, HEX or ROX, the quench group is selected from BHQ or TAMARA.

**Table 5: Reaction system for fluorescent quantitative PCR (20µl/tube)**

| reagent | amount (µl/tube) |
|---|---|
| double-distilled water | 9.9 |
| 10×buffer (free of Mg²⁺) | 2 |
| MgCl₂ (25mM) | 3 |
| dNTP (10 mM) | 0.5 |
| upstream primer (25µM) | 0.5 |
| downstream primer (25µM) | 0.5 |
| fluorescent probe (25µM) | 0.2 |
| Taq enzyme | 0.4 |
| DNA template | 3 |
| total volume | 20 |

For detecting the mutations in BRAF Codon 600, it needs to prepare two systems, in which all reagents are same except the probes. Specifically, for detecting BRAF Codon 600 wild-type genes, it needs to add BRAF-W-1 (SEQ ID NO: 9) or BRAF-W-2 (SEQ ID NO: 10) probes into the system; for detecting BRAF Codon 600 GTG**→**GAG mutant gene, it needs to add BRAF-M-1 (SEQ ID NO: 11) or BRAF-M-2 (SEQ ID NO: 12) probes into the system.

**Table 6: Probes**

| name | sequence | |
|---|---|---|
| BRAF-W-1 | CCA TCG AGA TTT CAC TGT AG | (SEQ ID NO:9) |
| BRAF-W-2 | CCATCGAGATTTCACTGTAGCTAG ACCA | (SEQ ID NO:10) |
| BRAF-M-1 | CCA TCG AGA TTT CTC TGT AG | (SEQ ID NO:11) |
| BRAF-M-2 | CCATCGAGATTTCTCTGTAGCTAGA CCA | (SEQ ID NO:12) |

**Table 7: Amplification condition**

| steps | Cycles | temperature and time |
|---|---|---|
| step 1 | 1 | 95 °C, 1-5minutes |
| step 2 | 30-45 | 95°C, 10-15 seconds; 55-65°C (collect fluorescent), 30-60 seconds |

### 3. Drawing the standard curve

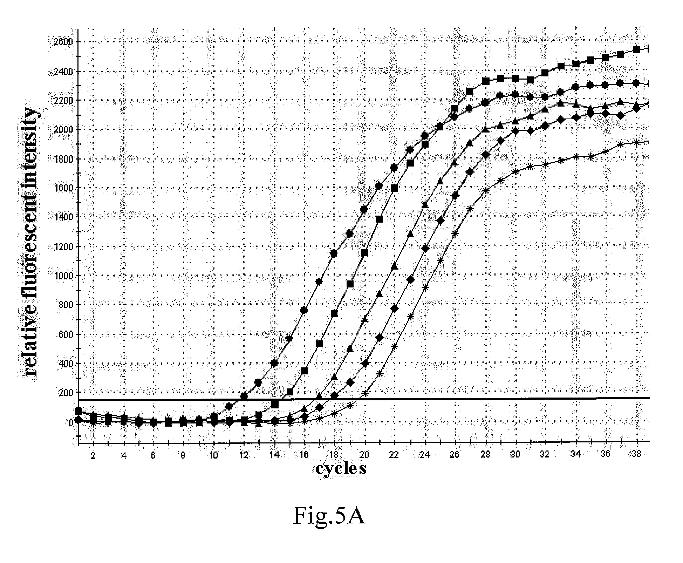

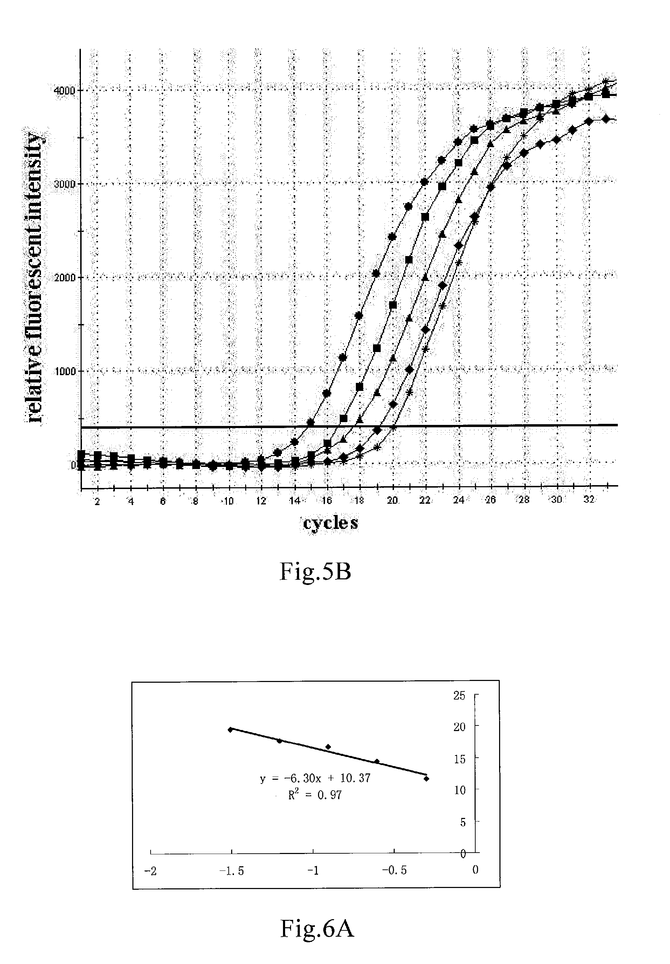

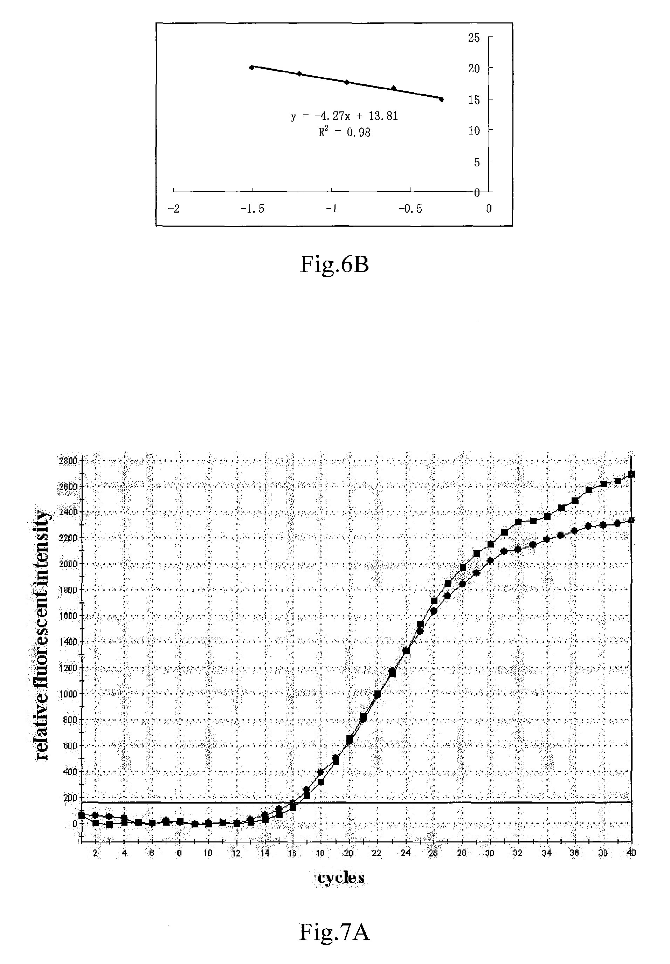

The standard curve is drawn based on the CT values obtained from the standard in Step 3. Figure 5 shows the amplification curve of plasmid standard, in which the five rising curves represent, from left to right, the amplification curve of the plasmid standard with the dilute ratio of 0.5ng/µl, 0.25ng/µl, 0.125ng/µl, 0.0625ng/µl and 0.03125ng/µl respectively. The horizontal axis represents cycle number, and the vertical axis represents fluorescent detection value. Accordingly, it is possible to draw the standard curve for calculation (Figure 6). In Figure 6, the horizontal axis represents the logarithm of copy number of the template, the vertical axis represents CT value, wherein the copy number of template=mass/(molecular weight)×6.02×10²³, the molecular weight of plasmid≈the number of bases ×324.5, or is calculated using the software DNAMAN. In the present experiment, the plasmid consists of pMD18-T carrier and an insert. Because the lengths of the insert are almost identical, the biggest difference only lies in twenties bases, which can be ignored with respect to the length of 2692bp for PMD18-T carrier. Therefore, the ratio of copies of wild-type to mutant plasmid standard ≈the ratio of weight.

### 4. Calculation of the ratio of specific BRAF mutation in a sample

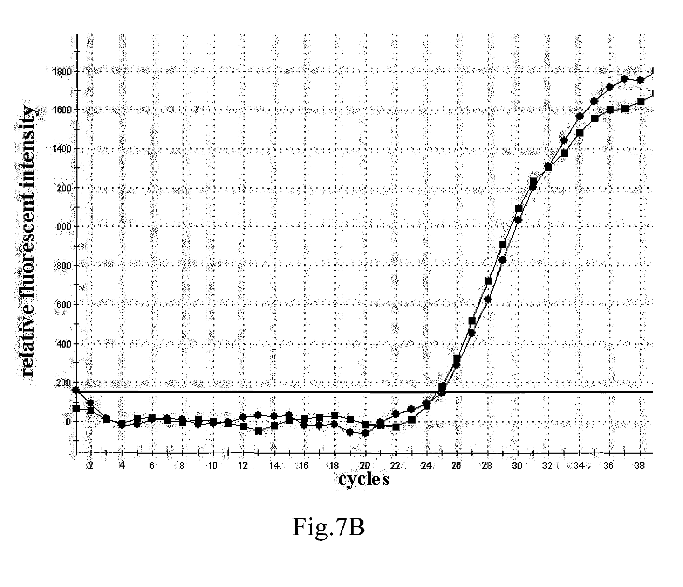

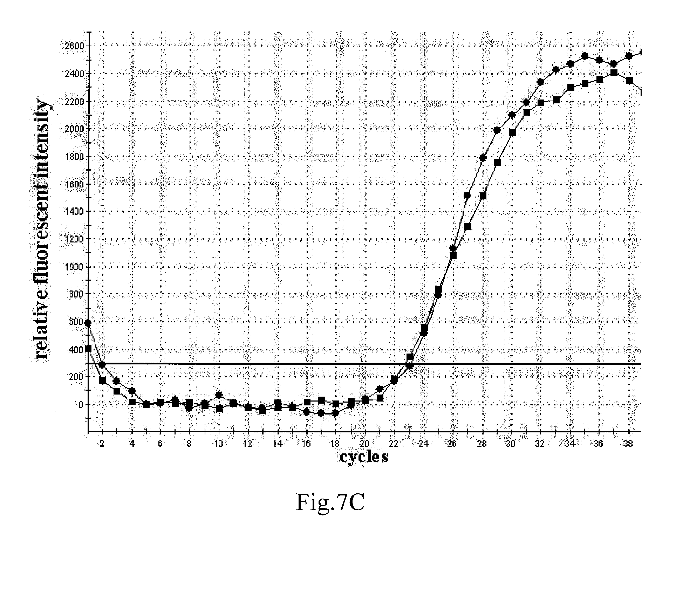

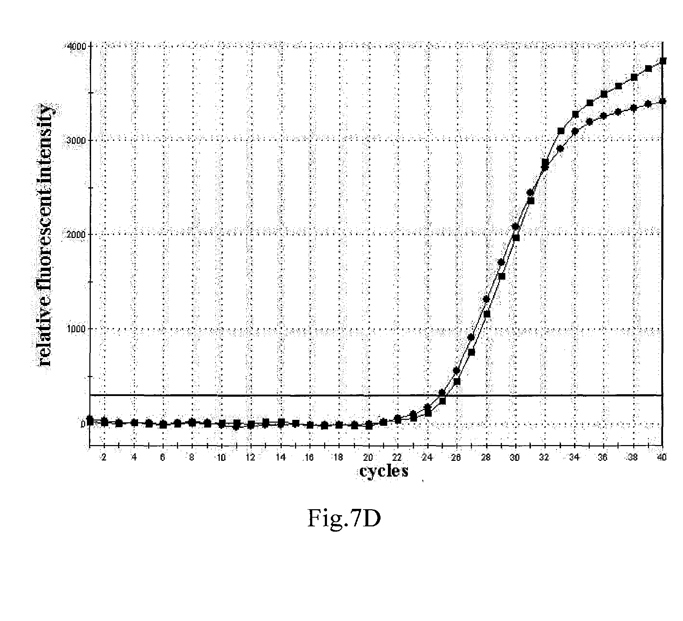

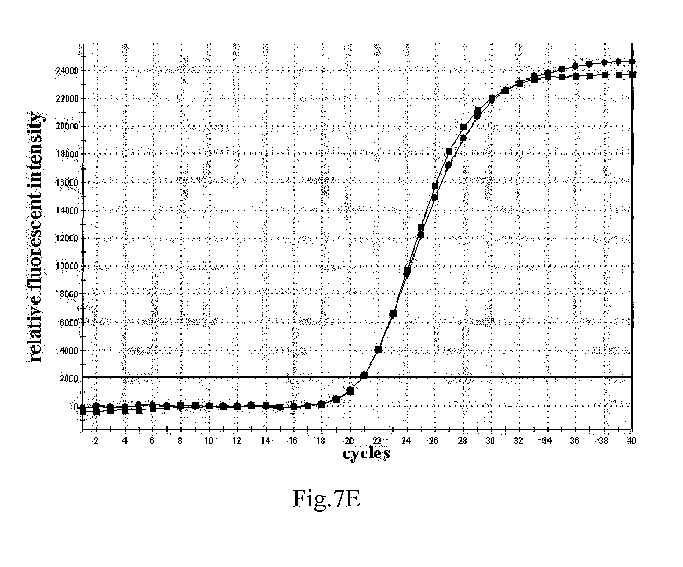

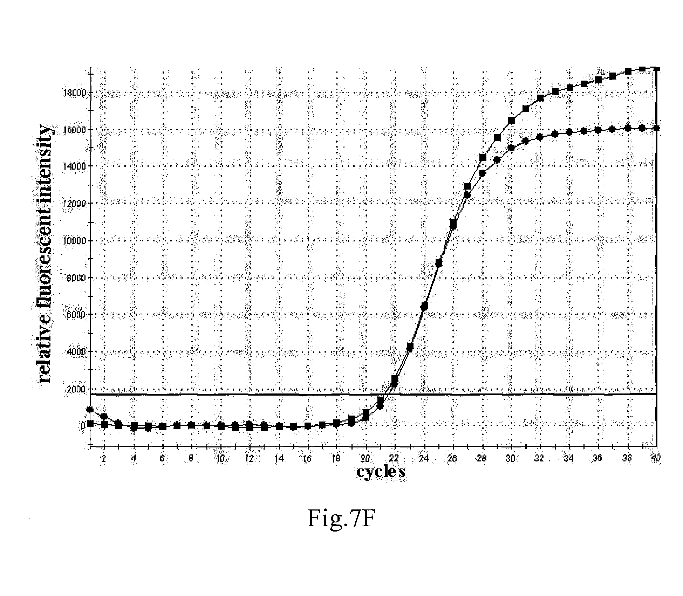

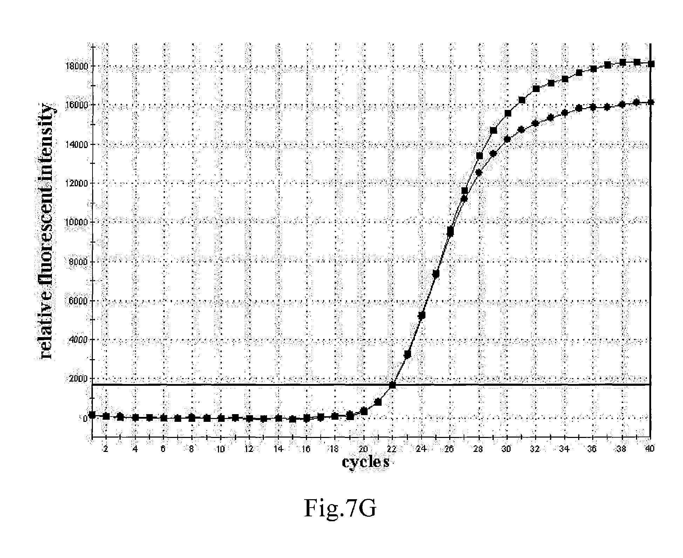

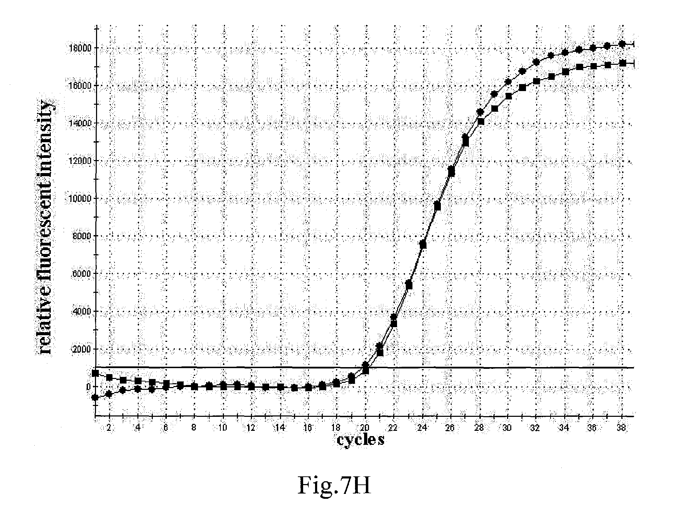

According to the standard curve, the copy numbers of wild-type and mutant genome DNA are calculated from the CT values of the sample. Then we obtain the ratio of mutant BRAF DNA to total BRAF DNA (wild-type plus all mutants at said site). As shown in Figure 7, the wild-type CT value of BRAF Codon 600 of a paraffin embedded lung cancer tissue sample is 16.20 (Fig.7A), whereas the value of GTG**→**GAG mutant is 24.95 (Fig.7B). According to each standard curve formula (Figure 6), we can calculate the copy numbers for them. We then obtain the ratio of the content of mutant to wild-type which was 1:50, and we estimate that about 2% BRAF gene in the tissue sample has GTG**→**GAG mutation in BRAF Codon 600.

### 5. Result of detection

In this Example, we detected the BRAF gene mutation in 80 cases of tissues, whole blood and cell line samples of thyroid carcinoma and colon cancer, and found that 8 cases had mutations. The mutation ratios, i.e. the ratio of mutant gene to non-mutant gene in those samples, can be seen in Table 8.

**Table 8: BRAF mutant cases**

| type of mutant samples | mutation cases | mutation ratio |
|---|---|---|
| KAT cell line | 1 | 40% |
| fresh thyroid carcinoma tissue | 2 | 15%, 25% |
| paraffin embedded colon cancer tissue | 4 | 20%, 25%, 50%, 55% |
| colon cancer whole blood | 1 | 30% |

## Claims

1. A PCR primer, **characterized in that** it binds with nucleotides within a sequence under a suitable PCR condition, said sequence having 200 bases and comprising a mutation site of the BRAF gene.

2. The primer according to claim 1, **characterized in that** said primer is consisted of upstream primer and downstream primer.

3. A probe for fluorescent quantitative PCR, **characterized in that** it specifically bind to the base sequence at BRAF gene mutant site under suitable PCR condition.

4. A probe according to claim 3, **characterized in that** said probe is linked to a fluorescence emitting group at its 5' end, and is linked to a fluorescence quenching group at its 3' end.

5. A plasmid containing BRAF gene wild-type sequence to be tested.

6. A plasmid according to claim 5, **characterized in that** the wild-type sequence of BRAF gene is SEQ ID NO:13.

7. A plasmid containing mutant sequence of BRAF gene to be tested.

8. The plasmid according to claim 7 **characterized in that** the BRAF gene mutant sequence contained in the plasmid is SEQ ID NO:14.

9. A quantitative detection kit for quantitatively detecting BRAF gene mutations, **characterized in that** it includes: the primers according to claim 1 or 2, the probes according to claim 3 or 4, and the plasmids according to claim 5 and/or 7.

10. A kit according to claim 9 **characterized in that** the ratio of said primer to probe is 2:1-10:1, and said primer comprises a forward primer and reverse primer in a ratio of 1:3-3:1.
